(19) Europäisches Patentamt
European Patent Office
Office européen des brevets

(11) Publication number: 0 034 481
B1

(12) EUROPEAN PATENT SPECIFICATION

(45) Date of publication of patent specification: 30.05.84

(21) Application number: 81300599.8

(22) Date of filing: 13.02.81

(51) Int. Cl.³: C 07 D 277/56, C 07 D 277/28, A 61 K 31/425, A 61 K 31/445

# (54) 2-Methyl-5-thiazole-methylamine and carboxamide derivatives.

(30) Priority: 14.02.80 GB 8004985

(43) Date of publication of application: 26.08.81 Bulletin 81/34

(45) Publication of the grant of the patent: 30.05.84 Bulletin 84/22

(84) Designated Contracting States: AT BE CH DE FR GB IT LI LU NL SE

(56) References cited:
EP-A-0 000 649
DE-A-1 645 928
FR-A-1 219 869
FR-A-1 481 686
FR-A-1 546 183
FR-A-1 601 733
FR-A-2 209 557
FR-A-2 221 138
FR-A-2 323 383
FR-A-2 361 111
FR-A-2 382 445
FR-M- 7 590
GB-A-1 241 589
GB-A-1 526 038
GB-A-2 020 662

The file contains technical information submitted after the application was filed and not included in this specification

(73) Proprietor: Grigg, Ronald Ernest
The Queen's University of Belfast Department of Chemistry David Keir Building
Belfast Northern Ireland (GB)

(72) Inventor: Grigg, Ronald Ernest
The Queen's University
of Belfast Dept. of Chemistry David Keir Building
Belfast Northern Ireland (GB)
Inventor: Amornraksa, Kitti
The Queen's University
of Belfast, Dept. of Chem. David Keir Building
Belfast Northern Ireland (GB)

(74) Representative: Perry, Robert Edward et al
GILL JENNINGS & EVERY 53-64 Chancery Lane
London WC2A 1HN (GB)

(56) References cited:
US-A-2 640 828
US-A-3 966 950
US-A-4 054 585
US-A-4 097 265
US-D- 359 768

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 034 481 B1

## Description

This invention relates to 5-thiazolemethylamine derivatives, their pharmacological utility, and to novel 5-thiazolecarboxamide derivatives.

British Patent Specification No. 1,526,038 discloses 5-thiazolemethylamine derivatives of the formula

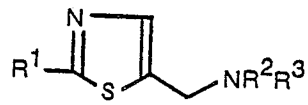

I

wherein $R^1$ is $C_{1-6}$ alkyl, and either $R^2$ and $R^3$ are each hydrogen, $C_{1-6}$ alkyl or $C_{1-6}$ hydroxyalkyl, or $NR^2R^3$ forms a heterocyclic ring containing one or two nitrogen atoms and bearing, if desired, a $C_{1-4}$ alkyl substituent, and acid addition salts thereof. Various examples of suitable heterocyclic rings are described. Syntheses of these compounds are described, starting from 2-alkyl-5-thiazolemethyl halides.

The compounds exemplified in British Patent Specification No. 1,526,038 are those of formula I in which $R^2$ and $R^3$ are each H, $CH_3$ or $HOCH_2CH_2$, and also 2-methyl-5-(piperidinomethyl)thiazole hemiethanedisulphonate and 2-methyl-5-(4-methylpiperazinomethyl)thiazole. The compounds are associated with anti-lipolytic activity, in that they reduce the amount of plasma free fatty acids, and hypoglycaemic activity. Pharmaceutical compositions are described, but the only specific examples of compositions and the only test results (given in the corresponding French Publications Nos. 2,323,383 and 2,372,678) relate to compounds in which $R^2$ and $R^3$ are each hydrogen, alkyl or hydroxyalkyl, except for a $LD_{50}$ test on the 4-methylpiperazino derivative.

U.S. Patent Specification No. 3,966,950 describes 2-alkyl-5-thiazolecarboxamides having $\alpha$-adrenolytic activity. French Patent Publication No. 2,209,557 discloses, as intermediates, 2-propyl-5-thiazolecarboxamides.

It has now been discovered that 2-methyl-5-(piperidinomethyl)thiazole and the novel compound 2-methyl-5-(pyrrolidinomethyl)thiazole and their acid addition salts, are of utility in stimulation of the central nervous system. These compounds can be prepared by reduction of the novel compounds 2-methyl-5-(piperidinocarbonyl)thiazole and 2-methyl-5-(pyrrolidinocarbonyl)thiazole, respectively.

The active compounds are within the scope of formula I. They are equivalent to that formula when $R^1$ is methyl and $NR^2R^3$ is piperidino or pyrrolidino, respectively.

For the reduction, the preferred reducing agent is lithium aluminium hydride. The reduction is usually conducted in a solvent such as ether.

The novel carboxamides may be prepared by reacting a 2-methyl-5-thiazolecarbonyl halide or 2-methyl-5-thiazolecarboxylate ester with piperidine or pyrrolidine at from ambient temperature to 100°C. The use of an alkyl, and most preferably the ethyl, ester is preferred. The reaction is preferably conducted in the presence of excess amine, as solvent, although substantially equimolar amounts of the halide/ester and amine may be used for reaction.

Suitable halides and esters for use as starting materials are known. Those which are not known may be made by procedures which are generally known, or analogous to those which have been described. A suitable halide may be prepared from the corresponding acid by reaction with thionyl chloride in the presence of pyridine. A suitable ester may be prepared by reacting thioacetamide with an alkyl, e.g. ethyl, $\alpha$-chloro-$\alpha$-formylacetate.

By way of alternative, the active compounds of the invention may be prepared by reduction of 2-methyl-5-thazolecarboxaldehyde with piperidine or pyrrolidine, in the presence of sodium cyanoborohydride. Suitable reaction conditions are in buffered solution at a pH of from 4 to 5, at from ambient temperature to 50°C.

In addition to their stimulatory effect on the cns, the active compounds of the invention have low peripheral activity. They can give a pleasurable sensation to the user, and may be self-administered for this purpose.

The compounds 2-methyl-5-(pyrrolidinomethyl)thiazole may be formulated as compositions for oral, parenteral or rectal administration. This compound or 2-methyl-5-(piperidinomethyl)thiazole may be formulated as a smoking product. They may be formulated in conventional manner, with a physiologically acceptable excipient or inert carrier which is administered to the subject with the active ingredient, or they may be formulated such that much of the composition remains outside the body, only the active ingredient and some other materials being taken into the body. For example, if the active ingredient is to be administered by inhalation, the composition may be in the form of a smoking product comprising an inert combustible substrate to which an appropriate amount of one or more of the compounds of the invention has been applied.

The compounds of the invention may be represented in a variety of tautomeric forms. These forms are within the scope of the invention.

The following Examples illustrate how compounds of the invention may be prepared.

Example 1

2-Methyl-5-(pyrrolidinocarbonyl)thiazole

A mixture of 15.42 g (0.0901 mole) of ethyl 2-methyl-5-thiazolecarboxylate and 12 g pyrrolidine was stirred at 120°C under a nitrogen atmosphere for 36 hours to give 12.91 g of the title compound, m.p. 71—2°C (78.6% yield with respect to the ester).

Example 2

2-Methyl-5-(piperidinocarbonyl)thiazole

In a manner parallel to that of Example 1, the title compound was prepared as white crystals, m.p. 68.7°C.

Example 3

2-Methyl-5-(pyrrolidinomethyl)thiazole

A solution of 2-methyl-5-(pyrrolidinocarbonyl)thiazole in dry ether was added dropwise to a suspension of lithium aluminium hydride and the mixture was stirred at room temperature to give the title compound as a colourless liquid, b.p. 61—62°C.

Example 4

2-Methyl-5-(piperidinomethyl)thiazole

Following the procedure of Example 3, the product of Example 2 was reduced to give the title compound as a colourless liquid, b.p. 65—66°C.

The products of the Examples are soluble in water and ethanol.

The products of the Examples have been tasted for their biological activity. Peripheral activity was determined by contraction of the guinea pig ileum/frog rectus while central nervous system activity was determined by adhesion to specific cell receptor surfaces. The results are given in the following Table.

| Compound of Example | Guinea Pig ileum test (% wrt nicotine) | Frog rectus test (% wrt nicotine) | Diazepam cell receptor adhesion (mM) |
|---|---|---|---|
| 1 | 0 | 0 | 0.55 |
| 2 | 0 | 0 | 0.49 |
| 3 | 16 | 300 | 4.4 |
| 4 | 9 | 140 | 1.0 |

$LD_{50}$ values for the compounds of Examples 3 and 4 are each 7—8 mg/kg.

**Claims**

1. 2-Methyl-5-(pyrrolidinomethyl)thiazole, or an acid addition salt thereof.
2. 2-Methyl-5-(pyrrolidinomethyl)thiazole, or an acid addition salt thereof, for use in a method of stimulation of the central nervous system.
3. 2-Methyl-5-(pyrrolidinocarbonyl)thiazole.
4. 2-Methyl-5-(piperidinocarbonyl)thiazole.
5. A process for preparing 2-methyl-5-(pyrrolidinomethyl)thiazole, which comprises reducing 2-methyl-5-(pyrrolidinocarbonyl)thiazole.
6. A process for preparing 2-methyl-5-(piperidinomethyl)thiazole, which comprises reducing 2-methyl-5-(piperidinocarbonyl)thiazole.
7. A smoking product which comprises an inert combustible substrate to which 2-methyl-5-(pyrrolidinomethyl)thiazole, or an acid addition salt thereof, has been applied.
8. A smoking product which comprises an inert combustible substrate to which 2-methyl-5-(piperidinomethyl)thiazole, or an acid addition salt thereof, has been applied.

**Patentansprüche**

1. 2-Methyl-5-(pyrrolidinomethyl)thiazol oder ein Säureadditionssalz hievon.
2. 2-Methyl-5-(pyrrolidinomethyl)thiazol oder ein Säureadditionssalz hievon zur Verwendung in einem Verfahren zur Stimulierung des Zentralnervensystems.
3. 2-Methyl-5-(pyrrolidinocarbonyl)thiazol.
4. 2-Methyl-5-(piperidinocarbonyl)thiazol.

5. Ein Verfahren zur Herstellung von 2-Methyl-5-(pyrrolidinomethyl)thiazol, welches das Reduzieren von 2-Methyl-5-(pyrrolidinocarbonyl)thiazol umfaßt.

6. Ein Verfahren zur Herstellung von 2-Methyl-5-(piperidinomethyl)thiazol, welches ein Reduzieren von 2-Methyl-5-(piperidinocarbonyl)thiazol umfaßt.

7. Ein Rauchartikel, der ein inertes brennbares Substrat umfaßt, auf welches 2-Methyl-5-(pyrrolidinomethyl)thiazol oder ein Säureadditionssalz hievon aufgebracht worden ist.

8. Ein Rauchartikel, der ein inertes brennbares Substrat umfaßtr, auf welches 2-Methyl-5-(piperidinomethyl)thiazol oder ein Säureadditionssalz hievon aufgebracht worden ist.

**Revendications**

1. Le 2-méthyl-5-(pyrrolidinométhyl)thiazole ou un de ses sels d'addition d'acides.

2. Le 2-méthyl-5-(pyrrolidinométhyl)thiazol ou un de ses sels d'addition d'acides, à utiliser dans un procédé de stimulation du système nerveux central.

3. Le 2-méthyl-5-(pyrrolidinocarbonyl)thiazole.

4. Le 2-méthyl-5-(pipéridinocarbonyl)thiazole.

5. Procédé de préparation du 2-méthyl-5-(pyrrolidinométhyl)thiazole, qui comprend la réduction du 2-méthyl-5-(pyrrolidinocarbonyl)thiazole.

6. Procédé de préparation du 2-méthyl-5-(pipéridinométhyl)thiazole, qui comprend la réduction du 2-méthyl-5-(pipéridinocarbonyl)thiazole.

7. Produit à fumer, qui comprend un substrat combustible inerte sur lequel a été appliqué du 2-méthyl-5-(pyrrolidinométhyl)thiazole ou un de ses sels d'addition d'acides.

8. Produit à fumer, qui comprend un substrat combustible inerte sur lequel a été appliqué du 2-méthyl-5-(pipéridinométhyl)thiazole ou un de ses sels d'addition d'acides.